# EUROPÄISCHE PATENTSCHRIFT

(11) **EP 2 298 727 B1**
(45) Veröffentlichungstag und Bekanntmachung des Hinweises auf die Patenterteilung: **04.03.2015**
(21) Anmeldenummer: 09011399.4
(22) Anmeldetag: 05.09.2009
(51) Int. Cl.: C07C 67/02, C07C 67/03, C07C 67/08, C10L 1/02, C10L 1/18, C11C 3/00, C10L 1/19, C12P 7/64

(54) **Verfahren zur Herstellung von Estern kurzkettiger Alkohole aus triglyceridreichen Ölen**
Method for producing esters of short-chains alcohols from triglyceride-rich oils
Procédé de fabrication d'esters d'alcools à chaîne courte à partir d'huiles riches en triglycérides

(43) Veröffentlichungstag der Anmeldung: 23.03.2011
(73) Patentinhaber: Cognis IP Management GmbH, 40589 Düsseldorf (DE)
(72) Erfinder: Kempers, Peter, 41189 Mönchengladbach (DE); Schörken, Ulrich, 40591 Düsseldorf (DE); Gutsche, Bernhard, 40724 Hilden (DE); Wolf, Thomas, 42781 Haan (DE); Dubreucq, Prof. Dr. Eric, 34000 Montpellier (FR); Moreau, Benoit, 7022 Hyon (BE)
(74) Vertreter: Popp, Andreas

(56) Entgegenhaltungen:
- EP-A1- 2 050 823
- EP-A2- 1 942 095
- WO-A1-03/010323
- WO-A2-2006/133698
- DE-A1-102005 002 700
- US-A1- 2007 277 432
- HAAS ET AL: "Combined Nonenzymatic-Enzymatic Method for the Synthesis of Simple Alkyl Fatty Acid Esters from Soapstock" JOURNAL OF THE AMERICAN OIL CHEMISTS' SOCIETY, SPRINGER, BERLIN, DE, Bd. 73, Nr. 11, 1. Januar 1997 (1997-01-01), Seiten 1393-1401, XP002147540 ISSN: 0003-021X

## Beschreibung

Die Erfindung befindet sich auf dem Gebiet Biotechnologie und betrifft ein enzymatisches Verfahren zur Herstellung von Fettsäureestern auf Basis von natürlichen Fetten und Ölen, speziell so genanntem "Biodiesel".

Ester pflanzlicher Öle werden in der Regel durch Niederdruck-Umesterung mit den entsprechenden Alkoholen, z.B. Methanol oder Ethanol, hergestellt. Diese Reaktion findet üblicherweise in Gegenwart alkalischer Katalysatoren statt. Von großtechnischer Bedeutung sind in erster Linie Kaliumhydroxid, Natriumhydroxid und Alkoholate. Während die Alkalimetallhydroxide häufig in der Literatur, in Patenten und Herstellanleitungen im Zusammenhang mit der Herstellung von speziellen Fettsäurealkylestergemischen, dem so genannten "Biodiesel", Erwähnung finden, sind Hinweise auf Alkoholate eher selten zu finden. Gleichwohl arbeiten die größten Biodiesel-Anlagen in Europa genau mit diesen Produkten. Die großtechnischen Verfahren des Stands der Technik werden dabei sowohl kontinuierlich als auch diskontinuierlich durchgeführt. Nach der Reaktion wird das Produkt neutralisiert, gewaschen und das überschüssige Methanol entfernt. Ferner wird das Glycerin abgetrennt und je nach Reinheit und späterer Anwendung weiter aufgearbeitet. Stark säurehaltige Öle, wie z.B. Kokos- oder Palmkernöl müssen dabei aufwendig raffiniert werden, da der Gehalt an freien Fettsäuren mit dem Katalysator zu Seife reagiert.

Eine Variante zur Herstellung von Biodiesel stellt die Hochdruck-Umesterung von pflanzlichen Ölen und tierischen Fetten in Gegenwart von Metallkatalysatoren dar, die bei hohem Druck und hoher Temperatur betrieben wird. Dabei können auch säurehaltige Öle eingesetzt werden, solche Verfahren sind jedoch sehr energieintensiv und benötigen einen hohen Überschuss an Methanol. Die aufwendige Methanol-Aufarbeitung trägt ebenfalls dazu bei, dass diese Verfahren sehr kostenintensiv sind.

Verfahren, die ganz oder teilweise Enzyme als Katalysatoren nutzen sind aus dem Stand der Technik ebenfalls bekannt. Problematisch ist jedoch, dass sich die bisher bekannten und beschriebenen Verfahren aus ökonomischen Gründen nicht realisieren lassen. Aus Eur. J. Lipid Sci. Technol. 110, 692-700 (2008) ist beispielsweise ein dreistufiges kontinuierliches Verfahren zur Biodiesel-Herstellung bekannt, bei dem immobilisierter Katalysator mit Lipase aus *Candida antarctica* B (Novozym 435) verwendet wird. Methanol wird zudosiert, da eine zu hohe Alkoholkonzentration das Enzym schädigen könnte. Die Kosten für ein solches Verfahren hängen stark von der Standzeit des Katalysators ab und stellen insbesondere beim Einsatz von rohen Kokos- oder Palmkernölen den kostenrelevanten kritischen Faktor dar. In J Am Oil Chem Soc. 84, 1015-1021 (2007) wird ebenfalls ein mehrstufiges Verfahren zur Hydrolyse und anschließender Veresterung von sauren Ölen beschrieben. Die hier in der ersten Stufe eingesetzte Lipase aus *Candida rugosa* zur Hydrolyse von Triglyceriden ist jedoch zu teuer, um sie für ein großtechnisch realisierbares Verfahren einzusetzen.

Die objektive Aufgabe der Erfindung war demnach, ein Verfahren zur enzymatischen Herstellung von Alkylestern bereitzustellen, welches kurzkettige Alkohole zum Einsatz bringt. Zudem war die Wiederverwendung von nicht-umgesetzten Einsatzstoffen erwünscht.

Gegenstand der Erfindung ist ein Verfahren zur Herstellung von Fettsäureestern kurzkettiger Alkohole, bei dem man
(i) pflanzliche Öle oder tierische Fette in Gegenwart von Enzymen, ausgewählt aus der Gruppe von Lipasen, Esterasen, Acyltransferasen und deren Gemischen, einer partiellen Umesterung und Hydrolyse mit wässrigem aliphatischem geradkettigem oder verzweigtem Ethanol, Ethanol, Propanol oder Butanol sowie deren Gemischen unterwirft und
(ii) anschließend das so erhaltene Reaktionsprodukt mit wasserfreiem aliphatischem geradkettigem oder verzweigtem Methanol, Ethanol, Propanol oder Butanol sowie deren Gemischen umestert bzw. verestert
wobei der abgetrennte nicht umgesetzte Alkohol aus Schritt (ii) im ersten Reaktionsschritt ohne weitere Reinigung wieder eingesetzt wird.

Überraschenderweise wurde gefunden, dass man zur Umesterung direkt saure Öle und selbst Abfallfette einsetzen kann, ohne eine vorherige Raffination durchführen zu müssen. Da das Verfahren bei Normaldruck arbeitet, sind weder aufwendige Investitionen für Druckanlagen erforderlich noch fallen hohe Energiekosten an, was zur Wirtschaftlichkeit des Verfahrens ebenfalls stark beiträgt. Außerdem können die wasserhaltigen Alkohole aus dem zweiten Reaktionsschritt direkt und ohne Aufarbeitung rückgeführt und wieder in den ersten Schritt eingespeist werden.

### Fettsäurekomponenten

In einer ersten Ausgestaltung der Erfindung werden als Fettsäurekomponenten triglyceridreiche pflanzliche Öle oder tierische Fette eingesetzt, ausgewählt aus der Gruppe, von Kokosöl, Palmöl, Palmkernöl, Sonnenblumenöl, Distelöl, Sojaöl, Rapsöl, Olivenöl, Reisöl, Cupheaöl, Schweineschmalz, Rindertalg und Fischöl sowie deren Gemischen. Alternativ - und wegen der großen Verfügbarkeit und des geringen Preises bevorzugt - ist der Einsatz von Abfallfetten, wie z.B. Frittierfette, glyceridhaltige Rückstände der ölverarbeitenden Industrie, die dem Fachmann unter den Bezeichnungen Crude Tall Oil, Vegetable Oil Distillates oder Soapstock bekannt sind.

### Alkoholkampanenten

Geeignete Alkoholkomponenten sind primäre oder sekundäre, geradkettige oder verzweigte Alkohole, wie Methanol, Ethanol, sowie die isomeren Propanole, und Butanole, sowie deren Gemische, insbesondere Gemische von Methanol und Ethanol im Volumenverhältnis 20:80 bis 80:20 und besonders bevorzugt ungefähr 50:50. In einer besonders bevorzugten Ausführungsform werden wieder Abfallprodukte eingesetzt, speziell Fuselöle aus der Ethanolfermentation als Alkoholmischung.

### Enzyme

Für die Umesterung eignen sich ganz besonders Enzyme, die ausgewählt sind aus der Gruppe, die gebildet wird von Lipasen, Esterasen und Acyltransferasen sowie deren Gemischen. Als Lipasen eignet sich beispielsweise *Thermomyces lanuginosus* oder Lipasen mit mehr als 50%iger Homologie zu dieser Spezies. Als Acyltransferase können vorzugsweise CpLip2 aus *Candida parapsilosis* oder Enzyme mit >30%iger Homologie zu dieser eingesetzt werden. Besonders bevorzugt sind Kombinationen von Lipasen und Acyltransferasen, speziell im Gewichtsverhältnis von etwa 1:1. Die Enzyme können in freier Form oder auf einem inerten Trägermaterial immobilisiert verwendet werden. Werden freie Enzyme eingesetzt, so hat es sich als vorteilhaft erwiesen, diese nach Beendigung der Reaktionsstufe (i) und/oder (ii) über die Abtrennung der alkoholischen Wasserphase aus der Reaktion zu entfernen und dann zusammen mit der Wasserphase wieder zu verwenden. Beim Einsatz von immobilisierten Enzymen empfiehlt es sich, diese nach Beendigung der Reaktionsstufe (i) und/oder (ii) vorzugsweise über ein im Reaktor integriertes Sieb abzufiltrieren und anschließend sowohl die alkoholische Wasserphase als auch die immobilisierten Enzyme ebenfalls wieder zu verwenden.

### Reaktionsführung

### Stufe i: Enzymatische Umesterung bzw. Hydrolyse

Die enzymatische Umesterung bzw. Hydrolyse der Fette und Öle kann batch-weise erfolgen, bevorzugt ist jedoch eine kontinuierliche Fahrweise, wobei diese vorteilhafterweise eine bis drei Retentionsstufen umfasst. Ein wesentlicher Vorteil des Verfahrens besteht dabei wie gesagt darin, dass unmittelbar mit wässrigen Alkoholen gearbeitet werden kann, wobei der Wasser:Alkoholgehalt im Bereich von ungefähr 10:90 bis 90:10, vorzugsweise ungefähr 25:75 bis 75:25 und insbesondere ungefähr 40:60 bis 60:40 Gewichtsteilen liegen kann. Die Reaktionsbedingungen orientieren sich schwerpunktmäßig an den optimalen Bedingungen, unter denen die Enzyme aktiv sind. Für die Temperatur bedeutet dies einen Bereich von 15 bis 45 und vorzugsweise ungefähr 30 °C, wobei es sich als vorteilhaft erwiesen hat, zur Beschleunigung der Acylmigration die Mischung zwischen den Schritten (i) und (ii) für einen Zeitraum von mindestens 5 und maximal 20 min auf 80 bis 100°C zu erwärmen. Die Umesterung bzw. Hydrolyse kann dabei bei Normaldruck durchgeführt werden. Falls gewünscht, kann die Ölphase zwischen den Stufen (i) und (ii) getrocknet werden.

### Stufe ii: Umsetzung mit kurzkettigen Alkoholen

Auch die Umsetzung der Reaktionsprodukte aus der ersten Stufe mit den kurzkettigen Alkoholen kann wieder batch-weise erfolgen, bevorzugt ist aber auch hier eine kontinuierliche Fahrweise, wobei diese wiederum eine bis drei Retentionsstufen umfassen kann. Die Umesterungsreaktion kann bei Temperaturen im Bereich ungefähr 35 bis 65 °C und bei Normaldruck durchgeführt werden. Als Katalysatoren werden hier ausschließlich oder in Kombination mit den Enzymen Metallseifen oder Säuren, vorzugsweise schwefelhaltige Säuren eingesetzt, wobei deren Konzentration bezogen auf die Einsatzstoffe typischerweise im Bereich von 0,1 bis 5 und vorzugsweise 0,5 bis 1 Gew.-% liegt.

### Stufe iii: Rückführung des Alkohols

Nach Abschluss der Reaktion wird nicht umgesetzter Alkohol ohne weitere Aufreinigung in die erste Verfahrensstufe zurückgeführt. Die Abtrennung der alkoholischen Wasserphase kann dabei über gravimetrische Phasentrennung oder einen Phasenabscheider erfolgen. Alternativ können Alkohol und Glycerin auch destillativ abgetrennt werden.

Die nach dem erfindungsgemäßen Verfahren erhältlichen Fettsäureester können in allen Gebieten Anwendung finden, für die diese Stoffe seit langem bekannt sind. Weisen die Ester einen Anteil von mehr als 30% langkettigen omega-3 Fettsäuren auf, wie sie beispielsweise auf Basis von Fischölen, Algenölen, mikrobiellen Ölen zugänglich sind, so eignen sie sich insbesondere als gesundheitsfördernde oder gesundheitserhaltende Nahrungsergänzungsmittel von hoher Farb-, Geruchs- und Geschmacksqualität oder als Vorstufe zur Herstellung von Nahrungsergänzungsmitteln, insbesondere zur Herstellung von Glyceriden über Resynthese mit Glycerin.

Ester auf Basis von pflanzlichen Ölen wie etwa Kokosöl, Palmkernöl, Babassuöl oder auch Cuphea, die einen Anteil von mehr als 50% mittelkettiger C₆-C₁₀-Fettsäuren enthalten, eignen sich, gegebenenfalls nach Fraktionierung, vor allem als Vorstufe zur Herstellung von Fettalkoholen, Tensiden oder Esterderivaten.

In einer besonderen Ausgestaltung der Erfindung werden die nach dem erfindungsgemäßen Verfahren hergestellten Ester als Treibstoff oder Treibstoffkomponente in Dieselmotoren eingesetzt.

### Beispiele

### Teil A

### Beispiele zur enzymatischen Reaktionen mit Thermomyces Lipase

### Beispiel 1: Enzymatische Umesterung von rohem Kokosöl mit wässrigem Ethanol

50g rohes Kokosöl und 70g einer wässrigen 14 Gew.-%igen Ethanollösung wurden in einen Kolben gegeben und unter Rühren auf 45°C aufgeheizt. Zum Ansatz wurden 2ml einer kommerziell erhältlichen Lipasepräparation (Lipolase 1001, *Thermomyces lanuginosus* Lipase) gegeben und die Reaktion bei 45°C inkubiert. Nach 3h, 6h und 24h wurden Proben entnommen und gaschromatographisch auf ihre Produktzusammensetzung hin untersucht. Die Ergebnisse sind in Tabelle 1 zusammengefasst.

**Tabelle 1**

| Produktzusammensetzung [GC-Flächen-%] | | | |
|---|---|---|---|
| Reaktionszeit [h] | Ethylester | Fettsäure | Glyceride |
| 3 | 35,0 | 31,6 | 33,4 |
| 6 | 37,7 | 30,4 | 31,9 |
| 24 | 53,5 | 34,8 | 11,7 |

Das Beispiel zeigt, dass die enzymatische Umesterung auch in stark verdünnten Systemen problemlos abläuft. Es erfolgt eine teilweise Umesterung zu Ethylestern und eine Hydrolyse zu Fettsäuren. Es wurden innerhalb von 24h bis zu 90% der Glyceride umgesetzt.

### Beispiel 2: Enzymatische Umesterung von MCT mit wässrigem Butanol

50g MCT ("Midchain-Triglyceride", ein synthetische hergestelltes Triglycerid mit einem Gehalt von >60% Capryl- und <40% Caprinsäure) und 105g einer wässrigen 14 Gew.-%igen Butanollösung wurden in einen Kolben gegeben und unter Rühren auf 45°C aufgeheizt. Zum Ansatz werden 2ml einer kommerziell erhältlichen Lipasepräparation (Lipolase 1001, *Thermomyces lanuginosus* Lipase) gegeben und die Reaktion wird bei 45°C inkubiert. Nach 3h, 6h und 24h werden Proben entnommen und gaschromatographisch auf ihre Produktzusammensetzung hin untersucht. Die Ergebnisse sind in Tabelle 2 zusammengefasst.

**Tabelle 2**

| Produktzusammensetzung [GC-Flächen-%] | | | | | |
|---|---|---|---|---|---|
| Reaktionszeit [h] | Butylester | Fettsäure | Monoglycerid | Diglycerid | Triglycerid |
| 3 | 38,5 | 24.5 | 17,7 | 22.3 | 5.1 |
| 6 | 44,8 | 17,6 | 13,4 | 19,0 | 4,7 |
| 24 | 57,9 | 24,5 | 2,5 | 9,0 | 5,6 |

Das Beispiel zeigt, dass die enzymatische Umesterung auch in stark wässrig verdünnten Systemen problemlos erfolgt. Die Umsetzung verläuft unter den gewählten Bedingungen innerhalb von 24h bis mehr als 80% Umsatz bei einem Überschuss an gebildeten Estern gegenüber freien Säuren.

### Beispiel 3: Einfluss von Ethanolkonzentration und Wasser auf Glycerinbildung, Säurebildung und Umesterungsgeschwindigkeit

Verschiedene Ansätze bestehend aus 40g Sonnenblumenöl und variablen Mengen Ethanol wurden mit jeweils 0,2g Lipolase bei Raumtemperatur einer Alkoholysereaktion unterzogen. Es wurden jeweils 25mg Na₂CO₃ zugesetzt. Der Gehalt an Glyceriden (MG=Monoglycerid, DG=Diglycerid, TG=Triglycerid) wurde wiederum gaschromatographisch analysiert; die Auswertung erfolgte über Flächenprozent. Der Glyceringehalt wurde ebenfalls gaschromatographisch analysiert und ist in nicht kalibrierten Flächenprozent angegeben. GC-Proben wurden für die Glycerinbestimmung nach 16h und für die Glyceridbestimmung nach 40h Reaktionszeit genommen; die Säurezahlen (SZ) wurden nach 16h bestimmt. Die Ergebnisse sind in Tabelle 3 zusammengefasst.

**Tabelle 3**

| Einsatzstoffe und Produktzusammensetzung [GC-Flächenprozent] | | | | | | | |
|---|---|---|---|---|---|---|---|
| Ansatz | Ethanol | Wasser | SZ | Glycerin | Ethylester | MG | MG:DG:TG |
| 1 | 15 g | 0,2 g | 2 | 1,5 | 62,2 | 29,2 | 86:14:0 |
| 2 | 30 g | 0,2 g | 1 | 0,3 | 34,5 | 11,4 | 18:35:47 |
| 3 | 15 g | 0,4 g | 3 | 2,4 | 64,3 | 11,4 | 86:14:0 |
| 4 | 30 g | 0,4 g | 1 | 0,5 | 58,9 | 30,6 | 77:23:0 |
| 5 | 15 g | 0,8 g | 5 | 2,8 | 64,7 | 25,8 | 87:13:0 |
| 6 | 30 g | 0,8 g | 2 | 1,1 | 62,4 | 32,2 | 92:8:0 |

Die Beispiele zeigen, dass die Erhöhung der relativen Wassermenge zu einer Erhöhung der Reaktionsgeschwindigkeit bei gleichzeitiger Erhöhung der gebildeten freien Säure führt.

### Beispiel 4 zum Vergleich: Enzymatische partielle Umesterung von Rapsöl im Pilotmaßstab

In einen 40001 Reaktor wurden insgesamt 1600kg raffiniertes Rapsöl, 640kg Ethanol, 600ml 1 M NaOH, 7l Wasser und 250.000 U Lipase (Esterase aus *Thermomyces,* Unit Angaben nach Hersteller) bezogen auf 1 kg Rapsöl gegeben. Der Ansatz wurde für 40h gerührt, danach unter weiterem Rühren auf 80°C aufgeheizt und für 2h bei 80°C gehalten, wobei der Behälter geschlossen blieb, so dass kein Ethanol entweichen konnte. Anschließend wurde auf 50°C abgekühlt, über einen Trommelfilter mit 10kg Celatom FW 14 filtriert und in Fässer abgefüllt. Es wurden 2200kg Produkt erhalten mit einer Ausbeute von 98% der Theorie. Das Produkt besaß die Zusammensetzung gemäß Tabelle 4:

**Tabelle 4**

| Produktzusammensetzung [GC-Flädhenprozent] | |
|---|---|
| Komponente | Anteil |
| Fettsäure | 1,0 |
| Ethylester | 35,8 |
| Monoglycerid | 21,1 |
| Diglycerid | 12,2 |
| Triglycerid | 2,0 |
| Glycerin | 0,25 |
| Wasser | 0,4 |
| Ethanol | ad 100 |

### Beispiel 5 zum Vergleich: Enzymatische partielle Umesterung von Rapsöl im Pilotmaßstab mit Aufarbeitung

In einen 4000l Reaktor wurden insgesamt 1600kg raffiniertes Rapsöl, 640kg Ethanol, 600ml 1M NaOH, 71 Wasser und 250.000 U Lipase (Esterase aus *Thermomyces,* Unit Angaben nach Hersteller) bezogen auf 1kg Rapsöl gegeben. Der Ansatz wurde für 44h gerührt, danach unter weiterem Rühren auf 120°C aufgeheizt. Es wurde Vakuum auf den Reaktor gelegt und das Ethanol - Wasser Gemisch abgezogen. Das Vakuum wurde dabei so lange langsam abgesenkt, bis kein Ethanol mehr aus dem Ansatz entwich. Anschließend wurde auf 50°C abgekühlt, über einen Trommelfilter mit 10kg Celatom FW 14 filtriert und in Fässer abgefüllt. Während der Umesterung werden Proben entnommen und analysiert. Die Ergebnisse sind in Tabelle 5a zusammengefasst.

**Tabelle 5a**

| Reaktionsverlauf [GC-Flächen-%] | | | | | | |
|---|---|---|---|---|---|---|
| T [h] | Glycerin | EE/FS | MG | DG | TG | SZ |
| 0 | 0 | 0 | 0 | 2,4 | 97.6 | 0,5 |
| 1 | 0 | 3.1 | 1 | 5,9 | 90 | 1,2 |
| 2 | 0 | 5,4 | 2,4 | 6,2 | 86 | 1,6 |
| 3 | 0 | 9,3 | 3,6 | 11,1 | 76 | 1,8 |
| 4 | 0 | 14,3 | 5,2 | 16,7 | 63,8 | 2,4 |
| 5 | 0 | 19,2 | 7 | 20,7 | 53,1 | 2,9 |
| 6 | 0 | 23.8 | 9,1 | 23,6 | 43.2 | 3,3 |
| 8 | 0 | 28,8 | 11,8 | 25,7 | 33,7 | 3,2 |
| 12 | 0 | 37,4 | 16,7 | 26,2 | 19,7 | 3,2 |
| 16 | 0 | 43,4 | 20,8 | 24,1 | 11,8 | 3,1 |
| 20 | 0,9 | 46,9 | 23,8 | 22,8 | 5,6 | 2,6 |
| 24 | 0 | 50,1 | 27,1 | 19,3 | 3,5 | 2,5 |
| 28 | 0 | 52,1 | 29,2 | 16,7 | 2 | 2,4 |
| 32 | 0 | 54,3 | 30,9 | 14,8 | 0 | 2,4 |
| 36 | 0.7 | 54,6 | 31,8 | 13 | 0 | 2,3 |
| 38 | 0,8 | 54,9 | 32.2 | 12,1 | 0 | 2,2 |
| 44 | 0,9 | 55,5 | 32,2 | 11,4 | 0 | 2,8 |

Es wurden 1742kg Produkt und 470kg Destillat entsprechend einer Ausbeute von 98% der Theorie erhalten. Die Produktzusammensetzung ist in Tabelle 5b wiedergegeben:

**Tabelle 5b**

| Produktzusammensetzung [GC-Flächenprozent] | |
|---|---|
| Komponente | Anteil |
| Fettsäure | 1,5 |
| Ethylester | 53,8 |
| Monoglycerid | 33,3 |
| Diglycerid | 12,1 |
| Triglycerid | < 1,0 |
| Glycerin | 0,5 |
| Wasser | < 0,1 |
| Ethanol | ad 100 |

### Beispiel 6: Enzymatische Umesterung von Fischöl mit Ethanol

In einen 21 Rührreaktor wurden 1kg Fischöl (Napro Pharma Fischöl 18/12), 100g Ethanol, 200g Wasser und 250µl 1 M NaOH vorgelegt. Unter Rühren wurden 10ml einer kommerziellen *Thermomyces* Lipase Präparation (Lipozym TL 100) zugegeben und der Ansatz bei 25°C inkubiert. Nach 3h und 6h wurden jeweils weitere 100g Ethanol zum Ansatz dosiert. Nach 5h und 24h wurden Proben entnommen und analysiert. Nach 24h wurde die wässrig/ethanolische Phase abgetrennt und das Reaktionsprodukt unter Vakuum getrocknet. Die Produktzusammensetzung ist in Tabelle 6 wiedergegeben:

**Tabelle 6**

| Produktzusammensetzung [GC-Flächenprozent] | |
|---|---|
| Komponente | Anteil |
| Fettsäuren + Ethylester | 57,3 |
| Monoglyceride | 18,8 |
| Diglyceride | 20,0 |
| Triglyceride | 3,7 |
| Säurezahl | 16,2 |

### Beispiel 7 zum Vergleich: Enzymatische Umesterung von Palmöl und Palmstearin mit Ethanol

80g Palmöl wurden in 80g Ethanol gelöst. Es wurden unter Rühren 1,5g Wasser und 0,5ml einer kommerziellen *Thermomyces* Lipase Präparation (Lipozym TL 100) zugegeben und der Ansatz bei 25°C inkubiert. Nach 3h und 6h wurden zunächst jeweils weitere 80g Palmöl und dann nach 24h weitere 0,25ml Lipozym TL 100 zugegeben. Nach 48h wurde die Reaktionsmischung analysiert. Der Versuch wurde unter Einsatz von Palmstearin wiederholt. Im ersten Fall wurden 50,6 Gew.-%, im zweiten Fall 49,4 Gew.-% (jeweils als GC-Flächen-%) erhalten.

### Beispiel 8 zum Vergleich: Enzymatische Umesterung von Rapsöl mit Methanol

40g Rapsöl, 1,5g Methanol, 10µl 1 M NaOH und 100µl Wasser wurden unter Rühren bei 20°C inkubiert. Zum Ansatz wurden 100µl einer kommerziellen *Thermomyces* Lipase Präparation (Lipozym TL 100) zugegeben und der Ansatz für weitere 48h unter Rühren inkubiert. Innerhalb der ersten 7 Stunden wurde stündlich jeweils 1g Methanol zum Ansatz dosiert. Nach 48h wurde die Zusammensetzung der Reaktionsmischung analysiert. Die Ergebnisse sind in Tabelle 8 zusammengefasst

**Tabelle 8**

| Produktzusammensetzung [GC-Flächenprozent] | |
|---|---|
| Komponente | Anteil |
| Methylester | 57,4 |
| Monoglycerid | 28,4 |
| Diglycerid | 8,7 |
| Triglycerid | 3,5 |
| Glycerin | 2,2 |
| Säurezahl | 1,8 |

### Beispiel 9: Umesterung von Kokosöl mit Methanol: Mehrmaliger Einsatz der Enzymphase

80g Kokosöl, 8g Methanol, 400µl 1M NaOH und 16g Wasser werden unter Rühren bei 30°C inkubiert. Zum Ansatz wurden 400µl einer kommerziellen *Thermomyces* Lipase Präparation (Lipozym TL 100) zugegeben und der Ansatz wurde für 2h unter Rühren inkubiert. Nach 2h wurde der Ansatz analysiert und die enzymhaltige Wasserphase in einem Scheidetrichter separiert. Nach 2h Separation wurde die enzymhaltige Wasserphase wieder mit 80g Kokosöl, 8g Methanol und 400µl 1M NaOH versetzt und erneut für 2h umgesetzt. Der Versuch wurde noch weitere dreimal unter den gleichen Bedingungen wiederholt. Die Ergebnisse sind in Tabelle 9 zusammengefasst.

**Tabelle 9**

| Produktzusammensetzung [GC-Flächenprozent] | | | | | | |
|---|---|---|---|---|---|---|
| Ansatz | SZ | T [h] | Fettsäuren | Methylester/ MG | Glyceride | Triglyceride |
| Kokosöl | 0 | 6,7 | 0,0 | 93,1 | 89,1 | 10,2 |

| Wiederholung | | | | | | |
|---|---|---|---|---|---|---|
| 1 | 2 | 48,5 | 48,5 | 28,2 | 14,2 | 29,1 |
| 2 | 2 | 51,9 | 51,9 | 27,6 | 13,3 | 28,2 |
| 3 | 2 | 48,5 | 48,5 | 32,9 | 15,0 | 28,5 |
| 4 | 2 | 50,6 | 50,6 | 32,3 | 15,2 | 25,4 |
| 5 | 2 | 46,2 | 46,2 | 42,4 | 23,0 | 17,4 |

Die Beispiele zeigen, dass bei der ersten Umsetzung ca. 50% Methylester/Monoglyceride gebildet werden. Die Separation der Wasserphase und deren erneuter Einsatz führt im zweiten Ansatz wiederum zu ca. 50% Methylester/Monoglycerid nach 2h Reaktionszeit. In diesem Versuch kann die enzymhaltige Wasserphase insgesamt fünfmal ohne erkennbaren Aktivitätsverlust eingesetzt werden, was zu einer deutlichen Kostenreduktion führt.

### Beispiel 10: Umesterung von Kokosöl mit Methanol im Pilotmaßstab

900kg Kokosöl, 90kg Methanol, 36kg Wasser, 4kg NaOH (1m) und 500 g einer kommerziellen *Thermomyces* Lipase Präparation (Lipozym TL 100) wurden bei 27°C für 24h unter Rühren inkubiert. Nach der Reaktion wurde die untere wässrige Phase absepariert und das Produkt durch Aufheizen und Anlegen von Vakuum getrocknet. Die Zusammensetzung ist in Tabelle 10 wiedergegeben.

**Tabelle 10**

| Produktzusammensetzung [GC-Flächenprozent] | | | | |
|---|---|---|---|---|
| Reaktionszeit [h] | Säurezahl | Fettsäure | Methylester | Monoglyceride |
| 0 | 9,9 | 0.00 | 0.00 | 0,00 |
| 1 | 16,6 | 7,74 | 14,80 | 5,14 |
| 2 | 19,5 | 8,88 | 20,50 | 8,27 |
| 3 | 23,0 | 9,54 | 22,33 | 9,50 |
| 4 | 23,7 | 10,72 | 24,81 | 10,30 |
| 6 | 24,3 | 10,06 | 29,78 | 13,08 |
| 8 | 23,8 | 10,00 | 31,48 | 15,70 |
| 12 | 21,1 | 9,06 | 34,22 | 18,05 |
| 24 | 23,8 | 11,64 | 41,11 | 22,00 |

Die Beispiele zeigen, dass nach Separation und Trocknung ca. 1005kg umgeestertes Produkt mit ca. 41 % Methylester erhalten wird. Die aus Separation und Trocknung gewonnenen wässrigen Methanol-Phasen können für weitere wässrige enzymatische Umsetzungen eingesetzt werden.

### Teil B

### Umesterungen mit immobilisiertem Enzym

### Beispiel 11: Umesterung von Rapsöl mit Ethanol

Zu 25g Dowex Marathon WBA wurden 25g einer kommerziellen *Thermomyces* Lipase Präparation (Lipozym TL 100) gegeben und die Mischung bei 8 °C inkubiert. Das feuchte Trägermaterial wurde direkt zur Umesterung eingesetzt. Hierzu wurden 4kg Rapsöl und 1kg Ethanol in einem Reaktor vorgelegt und die immobilisierte Lipase unter Rühren zugegeben. Der Ansatz wurde für 40h bei Raumtemperatur inkubiert und anschließend analysiert. Die Ergebnisse sind in Tabelle 11 zusammengefasst.

**Tabelle 11**

| Produktzusammensetzung [GC-Flächenprozent] | |
|---|---|
| Komponente | Anteil |
| Ethylester | 52,1 |
| Fettsäure | 3,7 |
| Monoglycerid | 27,0 |
| Diglycerid | 14,1 |
| Triglycerid | < 1,0 |
| Glycerin | 2,9 |

### Beispiel 12: Umesterung von Rapsöl mit Butanol

Zu 25g Döwex Marathon WBA wurden 25g einer kommerziellen *Thermomyces* Lipase Präparation (Lipozym TL 100) gegeben und die Mischung bei 8 °C inkubiert. Das feuchte Trägermaterial wurde direkt zur Umesterung eingesetzt. Hierzu wurden 3,7kg Rapsöl und 1,3 kg Butanol in einem Reaktor vorgelegt und die immobilisierte Lipase unter Rühren zugegeben. Der Ansatz wurde für 40h bei Raumtemperatur inkubiert und anschließend analysiert. Die Ergebnisse sind in Tabelle 12 zusammengefasst.

**Tabelle 12**

| Produktzusammensetzung [GC-Flächenprozent] | |
|---|---|
| Komponente | Anteil |
| Ethylester | 52,9 |
| Fettsäure | 3,8 |
| Monoglycerid | 19,2 |
| Diglycerid | 15,5 |
| Triglycerid | 6,1 |
| Glycerin | 2,4 |

### Beispiel 13: Batch-Umesterung von Rapsöl mit Ethanol: Wiederverwendung des Immobilisats

Zu 1g Dowex Marathon WBA wurden 10g Wasser und 0,5g einer kommerziellen *Thermomyces* Lipase Präparation (Lipozym TL 100) gegeben. Das Gemisch wurde für eine Stunde unter Schütteln inkubiert. Anschließend wurde es abfiltriert und zweimal mit 10ml auf -20 °C gekühltem Ethanol gewaschen. Danach wurden in einem Erlenmeyerkolben 50g Rapsöl und 2,5g Ethanol und 25µl 1M NaOH vorgelegt. Dazu wurde das Immobilisat gegeben und der Ansatz unter Schütteln bei 20 °C inkubiert. Nach 2, 4 und 6h wurden jeweils weitere 2,5g Ethanol zum Ansatz gegeben. Nach 24h wurde die Reaktion beendet und das Immobilisat abfiltriert. Mit dem gleichen Immobilisat wurden drei weitere Reaktionen nach dem gleichen Schema durchgeführt. Der vierte Ansatz wurde analog zum ersten Ansatz analysiert. Die Ergebnisse sind in Tabelle 13 zusammengefasst:

**Tabelle 13**

| Produktzusammensetzung [GC-Flächenprozent] | | | | | | |
|---|---|---|---|---|---|---|
| Ansatz | SZ | Glycerin | Ethylester | Monoglycerid | Diglycerid | Triglycerid |
| 1 | 2 | 1,3 | 57,4 | 27 | 12 | 1,2 |
| 4 | 2,6 | 2,2 | 52,2 | 22,4 | 18,4 | 6 |

Nach 4 Ansätzen wurden noch über 50% Ethylester erhalten.

### Beispiel 14: Kontinuierliche Umesterung von Rapsöl mit Ethanol

Eine Glassäule der Firma Amersham Pharmacia mit einem Durchmesser von 2,6cm wurde mit 52,2g Lipozym TL IM (kommerziell erhältliche immobilisierte *Thermomyces* Lipase von Novozymes) gefüllt. Mit einer Flussrate von 0,1 Säulenvolumen pro Stunde wurde eine Mischung aus 20% Ethanol und 80% Sonnenblumenöl über die Säule gepumpt und die Zusammensetzung am Säulenausgang analysiert. Die Säule wurde über 15 Tage kontinuierlich betrieben. Die Ergebnisse sind in Tabelle 14 zusammengefasst

**Tabelle 14**

| Produktzusammensetzung [GC-Flächenprozent] | | | | |
|---|---|---|---|---|
| Zeit [d] | Ethylester | Monoglyceride | Diglyceride | Triglyceride |
| 5 | 55,2 | 25,5 | 19,4 | 4,0 |
| 10 | 52,6 | 21,5 | 20,5 | 5,4 |
| 15 | 37,8 | 16,8 | 28,7 | 16,2 |

### Beispiel 15 Kontinuierliche Umesterung von Rapsöl mit Ethanol

Nach 15 Tagen wurde die in Beispiel 14 beschriebene Säule mit einem Gemisch aus Ethanol und Sonnenblumenöl im Verhältnis 1:4 und mit zusätzlich 1 % Emulgator Eumulgin® B2 betrieben. Der Emulgator bewirkt eine bessere Mischung der Startkomponenten. Die Säule mit dem gebrauchten Enzymimmobilisat wurde für weitere 10 Tage kontinuierlich mit einer Flussrate von 0,1 Säulenvolumen pro Stunde betrieben. Die Ergebnisse sind in Tabelle 15 zusammengefasst:

**Tabelle 15**

| Produktzusammensetzung [GC-Flächenprozent] | | | | |
|---|---|---|---|---|
| **Zeit [d]** | **Ethylester** | **Monoglyceride** | **Diglyceride** | **Triglyceride** |
| 5 | 53,9 | 27,6 | 15,7 | 2,8 |
| 10 | 38,1 | 20,3 | 24,2 | 17,4 |

### Teil C

### Mehrstufige Umesterungen mit Thermomyces Lipase

### Beispiel 16: Mehrstufige Umsetzung mit thermischer Zwischenbehandlung

400g Kokosöl, 40g Methanol, 20g Wasser und 1,6g 1 M NaOH wurden in einem Reaktor vorgelegt. Unter Rühren wurden 0,2g einer kommerziellen *Thermomyces* Lipase Präparation (Lipozym TL 100) gegeben, das Gemisch für 24h inkubiert und anschließend analysiert (Stufe 1). Methanol und Wasser wurden über Destillation entfernt und die Ölphase für 1h auf 120°C erhitzt. Danach wurde die Ölphase erneut mit 40g Methanol, 20 g Wasser und 0,8g 1M NaOH versetzt und die Reaktion durch Zugabe von 0,2g gestartet. Nach 24 h wurde die Reaktion beendet und analysiert (Stufe 2). Die Abfolge aus Erhitzen und enzymatischer Reaktion wurde weitere zweimal wiederholt, danach wurde die Ölphase erneut analysiert (Stufe 3). Die Ergebnisse sind in Tabelle 16 zusammengefasst:

**Tabelle 16**

| Produktzusammensetzung [GC-Flächenprozent] | | | |
|---|---|---|---|
| | **Stufe 1** | **Stufe 2** | **Stufe 3** |
| Ethylester | 37,8 | 55,8 | 81,1 |
| Fettsäure | 10,8 | 7,4 | 5.0 |
| Summe | 48,6 | 63,2 | 86,1 |

Das Beispiel zeigt, dass durch das Erhitzen der Reaktionslösung eine Acylmigration katalysiert wird. Dadurch kann die Lipase aus *Thermomyces* weitere glycerid-gebundene Fettsäuren umsetzen.

### Beispiel 17: Mehrstufige Umsetzung mit alkalischer Zwischenbehandlung

400g Kokosöl, 40g Methanol, 20g Wasser und 1,6g 1M NaOH wurden in einem Reaktor vorgelegt. Unter Rühren wurden 0,2 g einer Kommerziellen *Thermomyces* Lipase Präparation (Lipozym TL 100) gegeben, das Gemisch für 24h inkubiert und anschließend analysiert (Stufe 1). 50g der Ölphase wurden mit 2,5g Natriumcarbonat versetzt und für 1h auf 60°C erhitzt. Das Natriumcarbonat wurde abfiltriert und die Ölphase mit 5g Methanol, 2,5g Wasser, 0,1g 1M NaOH versetzt und bei 22°C inkubiert. Die Reaktion wurde durch Zugabe von 25µl Lipolase gestartet; nach 24h wurde die Reaktion beendet und analysiert (Stufe 2). Die Ergebnisse sind in Tabelle 17 zusammengefasst:

**Tabelle 17**

| Produktzusammensetzung [GC-Flachenprozent] | | |
|---|---|---|
| | **Stufe 1** | **Stufe 2** |
| Ethylester | 39,2 | 80,7 |
| Fettsäure | 10,2 | 9,7 |
| Summe | 49,4 | 90,4 |

Das Beispiel zeigt, dass durch das Erwärmen der Reaktionslösung in Anwesenheit eines basischen Salzes ebenfalls eine Acylmigration katalysiert wird. Dadurch kann die Lipase aus *Thermomyces* weitere glycerid-gebundene Fettsäuren umsetzen.

### Teil D

### Stufe 1 - Enzymatische Reaktionen mit Acyltransferase und strukturell ähnlichen Enzymen (Candida antarctica Lipase Typ A)

### Beispiel 18: Umesterungen mit freiem Enzym - Umesterung mit Candida antarctica Lipase A

In zwei Flaschen wurden jeweils 20g Kokosöl, 2g Methanol, 4g (Ansatz 1) bzw. 10g (Ansatz 2) Wasser und 0,1 g Novocor ADL (kommerziell erhältliche *Candida antarctica* Lipase A, Novozymes) gegeben und die Ansätze bei 20 °C unter Rühren inkubiert. Nach 5h und 24h wurden jeweils weitere 2g Methanol zugegeben. Nach 120h wurden die Ansätze analysiert. Die Ergebnisse sind in Tabelle 18 zusammengefasst:

**Tabelle 18**

| Produktzusammensetzung [GC-Flächenprozent] | | | |
|---|---|---|---|
| **Ansatz** | **Methylester** | **Fettsäuren** | **Glyceride** |
| 1 | 73,1 | 8,6 | 18,3 |
| 2 | 70,8 | 12,5 | 16,7 |

### Beispiel 19: Partielle Umesterung mit immobilisierter Candida antarctica Lipase A

In drei Fläschchen wurden jeweils 3g Neues Sonnenblumenöl (ölsäurereiche Züchtung des Sonnenblumenöls), 0,15 g auf Accurel MP1000 immobilisierte *Candida Lipase* A, 0,1 g Wasser und kurzkettige Alkohole gegeben, wie in Tabelle 19 dargestellt. Die Ansätze wurden für 140h unter Schütteln bei 30 °C inkubiert und anschließend analysiert.

**Tabelle 19**

| Produktzusammensetzung [GC-Flächenprozent] | | | | | |
|---|---|---|---|---|---|
| **Ansatz** | **Alkohol** | **Ester + Fettsäure** | **Monoglycerid** | **Diglycerid** | **Triglycerid** |
| 1 | 0,6 g Methanol | 87,3 | 3,4 | 3,5 | 5,6 |
| 2 | 1,5 g Butanol | 81,5 | 7,7 | 5,2 | 3,3 |
| 3 | 1,2 g | 66,6 | 5,9 | 7,7 | 17,3 |

### Beispiel 20: Enzymkombination aus Thermomyces Lipase und Acyltransferase

23g Glyceridgemisch aus Beispiel 10 wurden mit 11g Puffer pH 6,5 (100mM Phosphatpuffer), 2,2g Methanol und 40 µl Acyltransferase-Extrakt (276 U) versetzt. Der Ansatz wurde für 24 h bei 30°C gerührt. Die Zusammensetzung ist in Tabelle 20 wiedergegeben.

**Tabelle 20**

| Produktzusammensetzung [GC-Flächenprozent] | | |
|---|---|---|
| | **Startgemisch** | **2. Stufe** |
| Methylester | 41,1 | 57,9 |
| Monoglyceride | 22,0 | 6,5 |
| Diglyceride | 20,0 | 13,5 |
| Triglyceride | 5,0 | 4,8 |
| Fettsäuren | 11,6 | 18,7 |

### Beispiel 21: Umesterungen mit freiem Enzym

345g rohes Kokosöl, 64,5g Methanol, 375g Phosphatpufferlösung 50mM pH 6,5 und 600µl aufgereinigter Enzymextrakt von Acyltransferase CpLIP2 wurden für 24h bei 30°C gerührt. Die Zusammensetzung ist in Tabelle 21 wiedergegeben.

**Tabelle 21**

| Produktzusammensetzung [GC-Flächenprozent] | | | | | |
|---|---|---|---|---|---|
| **Zeit [h]** | **Fettsäuren** | **Methylester** | **Monoglycerid** | **Diglycerid** | **Triglycerid** |
| 0 | 7.0 | 0 | 0 | 4,3 | 85,7 |
| 4 | 6,7 | 12,3 | 2,0 | 10,8 | 64,1 |
| 24 | 8,6 | 35,0 | 4,8 | 13,5 | 41,8 |

### Beispiel 22: Umesterungen mit immobilisiertem Enzym

1500U der Acyltransferase CpLIP2 wurden auf 5,6g Lewatit VPOC 1600 Adsorberharz immobilisiert und mittels Glutaraldehyd quervernetzt. Mit dem hergestellten Biokatalysator werden 180g rohes Kokosöl, 9,6g Methanol, 40,4g Phosphatpuffer (50 mM, pH 6,5) für 72h bei 30°C gerührt. Die Methanol-Konzentration in der Pufferlösung wurde durch Dosierung auf einem konstanten Niveau gehalten. Nach 72h Reaktionszeit wurden 75% Methylester und ca. 8% Triglyceride (bestimmt als GC-Flächenprozente) erhalten, es konnte keine nennenswerte Bildung von freien Fettsäuren nachgewiesen werden. Sowohl im rohen Öl als auch im Produkt wurden 7,5% freie Fettsäure nachgewiesen.

### Stufe 2 - Enzymatische Umesterung / Veresterung der Gemische aus Stufe 1 mit immobilisierter Candida antarctica B Lipase

### Beispiel 23: Umesterung des Partialglycerids aus Beispiel 5

Das Gemisch aus Beispiel 5 wurde mit 5% Ethanol versetzt und in einer mit 10g Accurel MP 1000 (1g/g mit *Candida antarctica* B lipase beladen) befüllen Säule bei 45°C mit einer Flussrate von 6-30g/h durch die Enzymsäule gepumpt. Die sich bildende Glycerinphase wurde separiert, die Ölphase erneut mit 5% Ethanol versetzt und nochmals durch die Enzymsäule gepumpt. Die Produktzusammensetzung ist in Tabelle 23 wiedergegeben:

**Tabelle 23**

| Produktzusammensetzung [GC-Flächenprozent] | | | |
|---|---|---|---|
| | **Startgemisch** | **Stufe 1** | **Stufe 2** |
| Ethylester | 55,5 | 85,0 | 93,0 |
| Monoglyceride | 32,2 | 6,1 | 5,2 |
| Diglyceride | 11,4 | 7,3 | 1,3 |
| Triglyceride | 0 | 0 | 0 |
| SZ [mg KOH/g] | 2,8 | 1,0 | 0,7 |

### Beispiel 24: Umesterung des Partialglycerids aus Beispiel 6

Das Gemisch aus Beispiel 6 wurde mit 100g Ethanol und 20g Novozym 435 (kommerziell erhältliche immobilisierte *Candida antarctica* B Lipase) versetzt und unter Rühren und Stickstoffbegasung bei 45°C für 24h inkubiert. Danach wurde das Gemisch unter Vakuum bei 60°C getrocknet und analysiert (Stufe 1). Es wurden erneut 100g Ethanol zugegeben, das Gemisch erneut für 24h inkubiert, dann getrocknet und analysiert (Stufe 2). Die Produktzusammensetzung ist in Tabelle 24 wiedergegeben:

**Tabelle 24**

| Produktzusammensetzung [GC-Flächenprozent] | | | |
|---|---|---|---|
| | **Startgemisch** | **Stufe 1** | **Stufe 2** |
| Fettsäuren + Ethylester | 57,3 | 81,5 | 92,3 |
| Monoglyceride | 18,8 | 3,5 | 4,0 |
| Diglyceride | 20,0 | 6,2 | 3.0 |
| Triglyceride | 3,7 | 8,8 | 0.6 |
| SZ [mg KOH/g] | 16,2 | 4,7 | 3,9 |

### Beispiel 25: Umesterung des Partialglycerids aus Beispiel 21

20g getrocknetes vorverestertes Gemisch aus Beispiel 21 wurden mit 1g Methanol, 0,5g Novozym 435 (immobilisierte Lipase aus *Candida antarctica B*) bei 45°C gerührt. Nach 6h Reaktionszeit wurde 1 g Methanol zudosiert. Die Produktzusammensetzung ist in Tabelle 25 wiedergegeben:

**Tabelle 25**

| Produktzusammensetzung [GC-Flächenprozent] | | | | |
|---|---|---|---|---|
| **Zeit [h]** | **Methylester** | **Mono/Diglycerid** | **Triglycerid** | **SZ [mg KOH/g]** |
| 0 | 40,8 | 27,5 | 38,2 | 12,7 |
| 6 | 70,5 | 2,4 | 27,1 | 1,7 |
| 24 | 86,7 | 1,5 | 11,8 | 1,3 |

### Stufe 2 - Chemische Umesterung / Veresterung der Gemische aus Stufe 1

### Beispiel 26: Basisch katalysierte Umesterung des Partialglycerids aus Beispiel 5 mit Ethanol

Es wurden 100g getrocknetes Partialglycerid aus Beispiel 5 mit 15g Ethanol und 10g Natriumethylat (21 Gew.-%ig) für 5h bei 80°C umgesetzt. Die resultierende Produktzusammensetzung ist in Tabelle 26 wiedergegeben:

**Tabelle 26**

| Produktzusammensetzung [GC-Flächenprozent] | | |
|---|---|---|
| **Zeit [h]** | **Methylester** | **Mono/Di/Triglycerid** |
| 0 | 53,3 | 44,8 |
| 5 | 88,0 | 9,8 |

### Beispiel 27 Saure Umesterung des säurehaltigen Partialglycerids aus Beispiel 10 mit Methanol

Es wurden 100g getrocknetes Partialglycerid aus Beispiel 10 mit 10g Methanol und 1g konz. Schwefelsäure für 5h bei 65°C umgesetzt. Die resultierende Produktzusammensetzung ist in Tabelle 26 wiedergegeben:

**Tabelle 26**

| Produktzusammensetzung [GC-Flächenprozent] | | | |
|---|---|---|---|
| **Zeit [h]** | **Fettsäuren** | **Methylester** | **Mono/Di/Triglycerid** |
| 0 | 11,1 | 41,1 | 47,5 |
| 5 | 0,1 | 97,1 | 2,8 |

## Patentansprüche

1. Verfahren zur Herstellung von Fettsäureestern kurzkettiger Alkohole, bei dem man
i. pflanzliche Öle oder tierische Fette in Gegenwart von Enzymen, ausgewählt aus der Gruppe von Lipasen, Esterasen, Acyltransferasen und deren Gemischen, einer partiellen Umesterung und Hydrolyse mit wässrigem aliphatischem geradkettigem oder verzweigtem Methanol, Ethanol, Propanol oder Butanol sowie deren Gemischen unterwirft und
ii. anschließend das so erhaltene Reaktionsprodukt mit wasserfreiem aliphatischem geradkettigem oder verzweigtem Methanol, Ethanol, Propanol oder Butanol sowie deren Gemischen umestert bzw. verestert
wobei nicht umgesetzter Alkohol aus Schritt (ii) ohne weitere Aufreinigung in die erste Verfahrensstufe zurückgeführt wird.

2. Verfahren nach Anspruch 1, **dadurch gekennzeichnet, dass** man pflanzliche Öle oder tierische Fette einsetzt, ausgewählt aus der Gruppe von Kokosöl, Palmöl, Palmkernöl, Sonnenblumenöl, Distelöl, Sojaöl, Rapsöl, Olivenöl, Reisöl, Schweineschmalz, Rindertalg und Fischöl sowie deren Gemischen.

3. Verfahren nach Anspruch 1, **dadurch gekennzeichnet, dass** man Abfallfette einsetzt, ausgewählt aus der Gruppe von Frittierfetten, glyceridhaltigen Rückständen der ölverarbeitenden Industrie, vorzugsweise Crude Tall Oil, Vegetable Oll, Distillates oder Soapstock.

4. Verfahren nach einem der Ansprüche 1 bis 3, **dadurch gekennzeichnet, dass** man den Reaktionsschritt (I) bei einem Wasseranteil von 10 bis 90 und einem Alkoholgehalt von 90 bis 10 Gewichtsteilen durchführt.

5. Verfahren nach einem der Ansprüche 1 bis 4, **dadurch gekennzeichnet, dass** man als Lipasen *Thermomyces lanuginosus oder* Lipasen mit mehr als 50 %iger Homologie zu dieser einsetzt.

6. Verfahren nach einem der Ansprüche 1 bis 5, **dadurch gekennzeichnet, dass** man als Acyltransferasen Cplip2 aus *Candida parapsilosis oder* Enzyme mit > 30 %iger Homologie zu dieser einsetzt.

7. Verfahren nach mindestens einem der Ansprüche 1 bis 6, **dadurch gekennzeichnet, dass** man Fuselöl aus der Ethanolfermentation als Alkoholmischung einsetzt.

8. Verfahren nach einem der Ansprüche 1 bis 6, **dadurch gekennzeichnet, dass** man die freien Enzyme nach Beendigung der Reaktionsstufe (i) und/oder (ii) über die Abtrennung der alkoholischen Wasserphase aus der Reaktion entfernt und zusammen mit der alkoholischen Wasserphase wieder verwendet.

9. Verfahren nach einem der Ansprüche 1 bis 7, **dadurch gekennzeichnet, dass** die Verfahrensschritte (i) und/oder (ii) als kontinuierliche Reaktionen in einer Reaktionssäule durchgeführt werden, wobei diese eine bis drei Retentionsstufen umfasst

10. Verfahren nach einem der Ansprüche 1 bis 8, **dadurch gekennzeichnet, dass** man in der Reaktionsstufe (Ii) entweder ausschließlich oder in Kombination mit den Enzymen Metanseifen oder Säuren, vorzugsweise schwefelhaltige Säuren als Katalysatoren einsetzt.

11. Verfahren nach einem der Ansprüche 1 bis 9, **dadurch gekennzeichnet, dass** man die Reaktionsstufe (i) bei Temperaturen im Bereich von 15 bis 45 °C durchführt.

12. Verfahren nach einem der Ansprüche 1 bis 10, **dadurch gekennzeichnet, dass** man die Reaktionsstufe (ii) bei Temperaturen im Bereich 25 bis 65 °C durchführt.

## Claims

1. A process for the preparation of fatty acid esters of short-chain alcohols, in which
i. vegetable oils or animal fats are subjected, in the presence of enzymes selected from the group of lipases, esterases, acyl transferases and mixtures thereof, to a partial transesterification and hydrolysis with aqueous aliphatic straight-chain or branched methanol, ethanol, propanol or butanol, and mixtures thereof and
ii. then the reaction product obtained in this way is transesterified or esterified with anhydrous aliphatic straight-chain or branched methanol, ethanol, propanol or butanol, and mixtures thereof
wherein unreacted alcohol from step (ii) is returned to the first process stage without further purification.

2. The process according to claim 1, wherein vegetable oils or animal fats are used selected from the group of coconut oil, palm oil, palm kernel oil, sunflower oil, safflower oil, soybean oil, rapeseed oil, olive oil, rice oil, pork fat, beef tallow and fish oil, and mixtures thereof.

3. The process according to claim 1, wherein waste fats are used selected from the group of fried fats, glyceride-containing residues from the oil-processing industry, preferably crude tall oil, vegetable oil, distillates or soap stock.

4. The process according to any one of claims 1 to 3, wherein the reaction step (i) is carried out with a water fraction of 10 to 90 and an alcohol content of 90 to 10 parts by weight.

5. The process according to any one of claims 1 to 4, wherein the lipases used are *Thermomyces lanuginosus* or lipases with more than 50% homology to this.

6. The process according to any one of claims 1 to 5, wherein the acyl transferases used are Cplip2 from *Candida parapsilosis* or enzymes with > 30% homology to this.

7. The process according to at least one of claims 1 to 6, wherein fusel oil from the ethanol fermentation is used as alcohol mixture.

8. The process according to any one of claims 1 to 6, wherein the free enzymes are removed from the reaction at the end of reaction stage (i) and/or (ii) via the separation of the alcoholic water phase and reused together with the alcoholic water phase.

9. The process according to any one of claims 1 to 7, wherein process steps (i) and/or (ii) are carried out as continuous reactions in a reaction column, where the latter comprises one to three retention stages.

10. The process according to any one of claims 1 to 8, wherein, in reaction stage (ii), metal soaps or acids, preferably sulfur-containing acids, are used as catalysts either exclusively or in combination with the enzymes.

11. The process according to any one of claims 1 to 9, wherein reaction stage (i) is carried out at temperatures in the range from 15 to 45°C.

12. The process according to any one of claims 1 to 10, wherein reaction stage (ii) is carried out at temperatures in the range 25 to 65°C.

## Revendications

1. Procédé pour la préparation d'esters d'acides gras d'alcools à courtes chaînes, dans lequel
i. on soumet des huiles végétales ou des graisses animales en présence d'enzymes, choisies dans le groupe des lipases, des estérases, des acyltransférases et leurs mélanges, à une transestérification et une hydrolyse partielles avec du méthanol, de l'éthanol, du propanol ou du butanol aqueux, aliphatique(s), linéaire(s) ou ramifié(s) ainsi que leurs mélanges et
ii. on transestérifie ou on estérifie ensuite le produit de réaction ainsi obtenu avec du méthanol, de l'éthanol, du propanol ou du butanol aqueux, aliphatique(s), linéaire(s) ou ramifié(s) ainsi que leurs mélanges
l'alcool non transformé de l'étape (ii) étant recyclé sans autre purification dans le premier étage de procédé.

2. Procédé selon la revendication 1, **caractérisé en ce qu'**on utilise des huiles végétales ou des graisses animales choisies dans le groupe formé par l'huile de coco, l'huile de palme, l'huile de palmiste, l'huile de tournesol, l'huile de carthame, l'huile de soja, l'huile de colza, l'huile d'olive, l'huile de riz, le saindoux, le suif de boeuf et l'huile de poisson ainsi que leurs mélanges.

3. Procédé selon la revendication 1, **caractérisé en ce qu'**on utilise des graisses usagées, choisies dans le groupe des graisses de friture, des résidus contenant des glycérides de l'industrie de transformation des huiles, de préférence le tallol brut, l'huile végétale, les distillats ou la pâte de neutralisation.

4. Procédé selon l'une quelconque des revendications 1 à 3, **caractérisé en ce qu'**on réalise l'étape de réaction (i) à une proportion d'eau de 10 à 90 et une teneur en alcool de 90 à 10 parties en poids.

5. Procédé selon l'une quelconque des revendications 1 à 4, **caractérisé en ce qu'**on utilise, comme lipases, Thermomyces lanuginosus ou des lipases présentant une homologie supérieure à 50% par rapport à celle-ci.

6. Procédé selon l'une quelconque des revendications 1 à 5, **caractérisé en ce qu'**on utilise, comme acyltransférases, Cplip2 de Candida parapsilosis ou des enzymes présentant > 30% d'homologie par rapport à celle-ci.

7. Procédé selon au moins l'une quelconque des revendications 1 à 6, **caractérisé en ce qu'**on utilise de l'huile de fusel provenant de la fermentation d'éthanol comme mélange d'alcools.

8. Procédé selon l'une quelconque des revendications 1 à 6, **caractérisé en ce qu'**on élimine les enzymes libres, après la fin de l'étage de réaction (i) et/ou (ii) via la séparation de la phase aqueuse alcoolique, de la réaction et on les utilise à nouveau ensemble avec la phase aqueuse alcoolique.

9. Procédé selon l'une quelconque des revendications 1 à 7, **caractérisé en ce que** les étapes de procédé (i) et/ou (ii) sont réalisées sous forme de réactions continues dans une colonne de réaction, celle-ci comprenant un à trois étages de rétention.

10. Procédé selon l'une quelconque des revendications 1 à 8, **caractérisé en ce qu'**on utilise, dans l'étage de réaction (ii), des savons métalliques ou des acides, de préférence des acides contenant du soufre, comme catalyseurs soit exclusivement, soit en combinaison avec les enzymes.

11. Procédé selon l'une quelconque des revendications 1 à 9, **caractérisé en ce qu'**on réalise l'étage de réaction (i) à des températures dans la plage de 15°C à 45°C.

12. Procédé selon l'une quelconque des revendications 1 à 10, **caractérisé en ce qu'**on réalise l'étage de réaction (ii) à des températures dans la plage de 25°C à 65°C.
